(19) Europäisches Patentamt
European Patent Office
Office européen des brevets

(11) **EP 4 726 369 A1**

(12) **DEMANDE DE BREVET EUROPÉEN**

(43) Date de publication:
**15.04.2026 Bulletin 2026/16**

(51) Classification Internationale des Brevets (IPC):
**G01N 21/47** *(2006.01)* **A61B 5/00** *(2006.01)*
**A61B 5/1455** *(2006.01)*

(21) Numéro de dépôt: **25207901.7**

(22) Date de dépôt: **10.10.2025**

(52) Classification Coopérative des Brevets (CPC):
**G01N 21/474; A61B 5/00; A61B 5/0059;
A61B 5/1455;** G01N 2021/4735; G01N 2201/0628

(84) Etats contractants désignés:
**AL AT BE BG CH CY CZ DE DK EE ES FI FR GB
GR HR HU IE IS IT LI LT LU LV MC ME MK MT NL
NO PL PT RO RS SE SI SK SM TR**
Etats d'extension désignés:
**BA**
Etats de validation désignés:
**GE KH LA MA MD TN**

(30) Priorité: **14.10.2024 FR 2411097**

(71) Demandeur: **Commissariat à l'Energie Atomique
et aux Energies
Alternatives
75015 Paris (FR)**

(72) Inventeurs:
• **RACINE, Benoit**
  **38054 GRENOBLE CEDEX 09 (FR)**
• **SUHM, Aurélien**
  **38054 GRENOBLE CEDEX 09 (FR)**
• **LAUGIER, Christelle**
  **38054 GRENOBLE CEDEX 09 (FR)**
• **RASCLE, Angélique**
  **38054 GRENOBLE CEDEX 09 (FR)**
• **ANDRE, Luc**
  **38054 GRENOBLE CEDEX 09 (FR)**

(74) Mandataire: **INNOV-GROUP
209 Avenue Berthelot
69007 Lyon (FR)**

(54) **SYSTÈME DE MESURE SPECTROSCOPIQUE À RÉFLECTANCE DIFFUSE D'UN MILIEU À ANALYSER**

(57)     Système de mesure spectroscopique à réflectance diffuse d'un milieu (M) à analyser, comportant :
- un substrat (1) ;
- un dispositif électroluminescent (2), agencé sur une première zone (Z1) d'une surface (10) du substrat (1), et configuré pour émettre un rayonnement lumineux ; le dispositif électroluminescent (2) possédant une surface de sortie (S) par laquelle est transmis le rayonnement lumineux vers le milieu (M) à analyser ;
- un premier photodétecteur (D1), agencé sur une partie de la surface de sortie (S) du dispositif électroluminescent (2) de manière à recevoir une partie du rayonnement lumineux et de sorte que la surface de sortie (S) conserve une zone libre (ZL) ;
- un deuxième photodétecteur (D2), agencé sur une deuxième zone (Z2) de la surface (10) du substrat (1) pour recevoir le rayonnement lumineux transmis par la zone libre (ZL) et réfléchi de manière diffuse par le milieu (M) à analyser.

**Fig. 5**

EP 4 726 369 A1

## Description

### Domaine technique

L'invention se rapporte au domaine technique de la spectroscopie à réflectance diffuse.

L'invention trouve notamment son application dans l'identification non-invasive de composants biochimiques (e.g. taux d'oxygénation, glycémie, taux d'hydratation etc.) dans un tissu biologique tel que la peau.

### État de l'art

Un système de mesure spectroscopique à réflectance diffuse connu de l'état de la technique, notamment du document WO 2023/094887 A1, comporte :

- un substrat, comprenant une surface destinée à être orientée vers un milieu à analyser, la surface présentant des première et deuxième zones adjacentes ;
- une source lumineuse, agencée sur la première zone de la surface du substrat, et configurée pour émettre un rayonnement lumineux dans une gamme de longueurs d'onde ;
- un coupleur de sortie (« *output coupler* » en langue anglaise), agencé sur la deuxième zone de la surface du substrat, et possédant une surface de sortie par laquelle est transmise une partie majoritaire (e.g. 99%) du rayonnement lumineux vers un milieu à analyser ;
- un ondemètre (« *wavemeter* » en langue anglaise), agencé sur la deuxième zone de la surface du substrat ;
- un photodétecteur, agencé sur la deuxième zone de la surface du substrat pour recevoir le rayonnement lumineux transmis par la surface de sortie du coupleur de sortie et réfléchi de manière diffuse par le milieu à analyser.

Le rayonnement lumineux émis par la source lumineuse est fractionné de sorte que :

(i) une partie majoritaire (e.g. 99%) du rayonnement lumineux émis est guidée vers le coupleur de sortie afin de transmettre la partie majoritaire du rayonnement lumineux au milieu à analyser ;
(ii) une partie minoritaire (e.g. 1%) du rayonnement lumineux émis est guidée vers l'ondemètre afin de déduire la puissance lumineuse émise par la source lumineuse.

La détermination du nombre de photons émis par la source lumineuse vers le milieu à analyser est essentielle afin d'obtenir une spectroscopie à réflectance diffuse significative.

Un tel système de mesure spectroscopique à réflectance diffuse de l'état de la technique n'est pas entièrement satisfaisant dans la mesure où la présence de guides d'ondes (vers le coupleur de sortie et vers l'ondemètre) complexifie la fabrication du système de mesure et introduit des pertes optiques importantes, réduisant ainsi l'efficacité du système (augmentation de l'énergie consommée). De plus, le coupleur de sortie et l'ondemètre occupent une aire substantielle de la deuxième zone de la surface du substrat, introduisant un certain encombrement latéral pouvant provoquer un inconfort lorsque le système de mesure est porté par un patient.

### Exposé de l'invention

L'invention vise à remédier en tout ou partie aux inconvénients précités. A cet effet, l'invention a pour objet un système de mesure spectroscopique à réflectance diffuse d'un milieu à analyser, comportant :

- un substrat, comprenant une surface destinée à être orientée vers le milieu à analyser, la surface présentant des première et deuxième zones adjacentes ;
- un dispositif électroluminescent, agencé sur la première zone de la surface du substrat, et configuré pour émettre un rayonnement lumineux dans une gamme de longueurs d'onde ; le dispositif électroluminescent possédant une surface de sortie par laquelle est transmis le rayonnement lumineux vers le milieu à analyser ;
- un premier photodétecteur, dit de référence, agencé sur une partie de la surface de sortie du dispositif électroluminescent de manière à recevoir une partie du rayonnement lumineux et de sorte que la surface de sortie conserve une zone libre ;
- un deuxième photodétecteur, dit de mesure, agencé sur la deuxième zone de la surface du substrat pour recevoir le rayonnement lumineux transmis par la zone libre de la surface de sortie du dispositif électroluminescent et réfléchi de manière diffuse par le milieu à analyser.

Ainsi, un tel système selon l'invention permet de s'affranchir de la présence de guides d'ondes par rapport à l'état de la technique. En effet, d'une part, il n'existe pas de coupleur de sortie puisque le rayonnement lumineux est transmis directement via la surface de sortie du dispositif électroluminescent. D'autre part, le photodétecteur de référence (permettant de déduire la puissance lumineuse émise par le dispositif électroluminescent) est agencé sur une partie de la surface de sortie du dispositif électroluminescent. De plus, il en résulte un encombrement latéral réduit par rapport à l'état de la technique en empilant le photodétecteur de référence sur le dispositif électroluminescent.

Le système selon l'invention peut comporter une ou plusieurs des caractéristiques suivantes.

Selon une caractéristique de l'invention, le dispositif électroluminescent comporte au moins une diode électroluminescente organique.

Ainsi, un avantage procuré par un tel dispositif électroluminescent est d'obtenir un angle d'émission large avec peu de pertes en luminance entre le centre et la périphérie, de sorte que la surface de sortie est homogène.

Selon une caractéristique de l'invention, le premier photodétecteur comporte au moins une photodiode organique.

Ainsi, un avantage procuré par un tel photodétecteur est de pouvoir être fabriqué aisément sur le dispositif électroluminescent, tout particulièrement lorsque le dispositif électroluminescent est organique.

Selon une caractéristique de l'invention, le deuxième photodétecteur comporte au moins une photodiode organique.

Ainsi, un avantage procuré est de pouvoir fabriquer aisément les premier et deuxième photodétecteurs de manière concomitante lorsque ceux-ci sont organiques.

Selon une caractéristique de l'invention, le dispositif électroluminescent comporte successivement :

- une première électrode, agencée sur la première zone de la surface du substrat ;
- un empilement de couches, comprenant une couche émissive configurée pour émettre le rayonnement lumineux dans la gamme de longueurs d'onde ;
- une seconde électrode, transparente dans la gamme de longueurs d'onde, et présentant une surface définissant la surface de sortie du dispositif électroluminescent.

La seconde électrode doit être transparente dans la gamme de longueurs d'onde afin de transmettre le rayonnement lumineux émis par la couche émissive. La première électrode est avantageusement réfléchissante dans la gamme de longueurs d'onde afin de réduire les pertes optiques. Ainsi, une contrainte est levée sur la nature du substrat qui peut ne pas être réfléchissant dans la gamme de longueurs d'onde pour réduire les pertes optiques. Toutefois, si la première électrode n'est pas réfléchissante dans la gamme de longueurs d'onde, il est alors possible d'utiliser un substrat réfléchissant dans la gamme de longueurs d'onde afin de réduire les pertes optiques.

Selon une caractéristique de l'invention, le premier photodétecteur comporte successivement :

- une première électrode, transparente dans la gamme de longueurs d'ondes, et agencée sur une partie de la surface de sortie du dispositif électroluminescent ;
- un empilement de couches, comprenant une couche absorbante configurée pour absorber le rayonnement lumineux dans la gamme de longueurs d'onde ;
- une seconde électrode, réfléchissante dans la gamme de longueurs d'onde.

La première électrode est transparente dans la gamme de longueurs d'onde afin que le rayonnement lumineux émis par le dispositif électroluminescent puisse atteindre la couche absorbante. La seconde électrode est réfléchissante dans la gamme de longueurs d'onde afin que le rayonnement lumineux résiduel, non-absorbé par la couche absorbante, ne se propage pas dans le milieu à analyser.

Selon une caractéristique de l'invention, la première électrode du premier photodétecteur est agencée sur une partie latérale de la surface de sortie.

Ainsi, un avantage procuré est de faciliter l'accès pour une connexion électrique du premier photodétecteur tout en optimisant la distribution du rayonnement lumineux vers le milieu à analyser.

Selon une caractéristique de l'invention :

- la surface de sortie possède une aire, notée « $A_S$ » ;
- la première électrode du premier photodétecteur occupe une aire, notée « $A_P$ », vérifiant :

$$A_P = \frac{A_S}{n}$$

où « $n$ » est un entier naturel supérieur ou égal à 2.

Ainsi, un avantage procuré est de faciliter la détermination du nombre de photons émis par le dispositif électroluminescent vers le milieu à analyser afin d'obtenir une spectroscopie à réflectance diffuse significative. Le nombre total de photons émis à un instant donné par le dispositif électroluminescent correspond à « $n$ » fois le nombre de photons détectés par le premier photodétecteur à l'instant donné. Le nombre de photons émis à un instant donné par le dispositif électroluminescent vers le milieu à analyser (i.e. transmis via la zone libre de la surface de sortie) correspond à « $n-1$ » fois le nombre de photons détectés par le premier photodétecteur à l'instant donné.

Selon une caractéristique de l'invention :

- l'aire de la surface de sortie est comprise entre 6400 $\mu m^2$ et 14400 $\mu m^2$, de préférence comprise entre 8100 $\mu m^2$ et 12100 $\mu m^2$ ;
- « $n$ » est égal à 2.

Ainsi, un avantage procuré par cette configuration géométrique est de s'affranchir d'un composant électronique de calcul arithmétique tout en conservant une distribution du rayonnement lumineux satisfaisante entre la zone libre (et donc le milieu à analyser) et le premier photodétecteur. En effet, le nombre de photons émis à un instant donné par le dispositif électroluminescent vers le milieu à analyser (i.e. transmis via la zone libre de la surface de sortie) correspond alors au nombre de photons détectés par le premier photodétecteur à l'instant donné.

Selon une caractéristique de l'invention, le substrat est réalisé dans un matériau réfléchissant dans la gamme de longueurs d'onde.

Ainsi, un avantage procuré est de lever une contrainte sur la première électrode du dispositif électroluminescent afin de réduire les pertes optiques. En effet, lorsque le substrat est réalisé dans un matériau réfléchissant dans la gamme de longueurs d'onde, la première électrode du dispositif électroluminescent peut ne pas être réfléchissante dans la gamme de longueurs d'onde.

L'invention a enfin pour objet un procédé de fabrication d'un système de mesure spectroscopique à réflectance diffuse d'un milieu à analyser, comportant les étapes :

a) utiliser un substrat, comprenant une surface destinée à être orientée vers le milieu à analyser, la surface présentant des première et deuxième zones adjacentes ;

b) former un dispositif électroluminescent sur la première zone de la surface du substrat, le dispositif électroluminescent étant configuré pour émettre un rayonnement lumineux dans une gamme de longueurs d'onde ; le dispositif électroluminescent possédant une surface de sortie par laquelle est transmis le rayonnement lumineux vers le milieu à analyser ;

c) former un premier photodétecteur, dit de référence, sur une partie de la surface de sortie du dispositif électroluminescent de manière à recevoir une partie du rayonnement lumineux et de sorte que la surface de sortie conserve une zone libre ;

d) former un deuxième photodétecteur, dit de mesure, sur la deuxième zone de la surface du substrat pour recevoir le rayonnement lumineux transmis par la zone libre de la surface de sortie du dispositif électroluminescent et réfléchi de manière diffuse par le milieu à analyser.

Ainsi, comme évoqué précédemment, un tel procédé selon l'invention permet de s'affranchir de la présence de guides d'ondes par rapport à l'état de la technique. En effet, d'une part, il n'existe pas de coupleur de sortie puisque le rayonnement lumineux est transmis directement via la surface de sortie du dispositif électroluminescent. D'autre part, le photodétecteur de référence (permettant de déduire la puissance lumineuse émise par le dispositif électroluminescent) est agencé sur une partie de la surface de sortie du dispositif électroluminescent. De plus, il en résulte un encombrement latéral réduit par rapport à l'état de la technique en empilant le photodétecteur de référence sur le dispositif électroluminescent.

Le procédé selon l'invention peut comporter une ou plusieurs des caractéristiques suivantes.

Selon une caractéristique de l'invention, les étapes c) et d) sont concomitantes.

Ainsi, un avantage procuré est de réduire la durée d'exécution du procédé.

Selon une caractéristique de l'invention, l'étape b) comporte les étapes :

$b_1$) former une première électrode sur la première zone de la surface du substrat ;

$b_2$) former un empilement de couches sur la première électrode, l'empilement comprenant une couche émissive configurée pour émettre le rayonnement lumineux dans la gamme de longueurs d'onde ;

$b_3$) former une seconde électrode, transparente dans la gamme de longueurs d'onde, sur l'empilement de couches, et présentant une surface définissant la surface de sortie du dispositif électroluminescent ;

procédé dans lequel les étapes c) et d) concomitantes comportent les étapes :

- former une première électrode sur une partie de la surface de sortie du dispositif électroluminescent ainsi que sur la deuxième zone de la surface du substrat ;
- former un empilement de couches, comprenant une couche absorbante configurée pour absorber le rayonnement lumineux dans la gamme de longueurs d'onde, sur la première électrode du premier photodétecteur ainsi que sur la première électrode du deuxième photodétecteur ;
- former une seconde électrode sur l'empilement de couches du premier photodétecteur ainsi que sur l'empilement de couches du deuxième photodétecteur.

Selon une caractéristique de l'invention, l'étape b) est précédée des étapes :

- former des premiers plots de contact, agencés sur la première zone de la surface du substrat pour une reprise de contact électrique de la seconde électrode du dispositif électroluminescent ;
- former des deuxièmes plots de contact, agencés sur la première zone de la surface du substrat pour une reprise de contact électrique de la seconde électrode du premier photodétecteur ;
- former des troisièmes plots de contact, agencés sur la deuxième zone de la surface du substrat pour une reprise de contact électrique de la seconde électrode du deuxième photodétecteur ;

procédé dans lequel :

- la seconde électrode du dispositif électroluminescent formée lors de l'étape $b_3$) s'étend sur la première zone de la surface du substrat de manière à être électriquement connectée aux premiers plots de contact ;

- la seconde électrode du premier photodétecteur et la seconde électrode du deuxième photodétecteur formées lors des étapes c) et d) concomitantes s'étendent respectivement sur les première et deuxième zones de la surface du substrat, de manière à être respectivement électriquement connectées aux deuxièmes et troisièmes plots de contact.

Ainsi, un avantage procuré est de faciliter la reprise de connexion électrique tout en limitant la durée d'exécution du procédé.

### *Définitions*

- Par « substrat », on entend un support physique autoporté, réalisé dans un matériau de base à partir duquel peuvent être formés un dispositif électroluminescent et un photodétecteur. Un substrat peut être une « tranche » (également dénommée « plaquette », « *wafer* » en langue anglaise) qui se présente généralement sous la forme d'un disque issu d'une découpe dans un lingot d'un matériau cristallin.
- Par « gamme de longueurs d'onde », on entend un intervalle de valeurs dans lequel la longueur d'onde du rayonnement lumineux émis par le dispositif électroluminescent est comprise. L'intervalle de valeurs peut tendre vers une valeur lorsque le rayonnement lumineux est monochromatique.
- Par « zone libre », on entend une zone de la surface de sortie du dispositif électroluminescent qui n'est pas recouverte par le premier photodétecteur (i.e. le photodétecteur de référence).
- Par « réfléchissant(e) », on entend que l'élément possède un coefficient de réflexion en intensité supérieur ou égal à 60%, de préférence supérieur ou égal à 80%, plus préférentiellement supérieur ou égal à 90%, moyenné dans la gamme de longueurs d'onde.
- Par « transparent(e) », on entend que l'élément possède un coefficient de transmission en intensité supérieur ou égal à 60%, de préférence supérieur ou égal à 80%, plus préférentiellement supérieur ou égal à 90%, moyenné dans la gamme de longueurs d'onde.

### Brève description des dessins

D'autres caractéristiques et avantages apparaîtront dans l'exposé détaillé de différents modes de réalisation de l'invention, l'exposé étant assorti d'exemples et de références aux dessins joints.

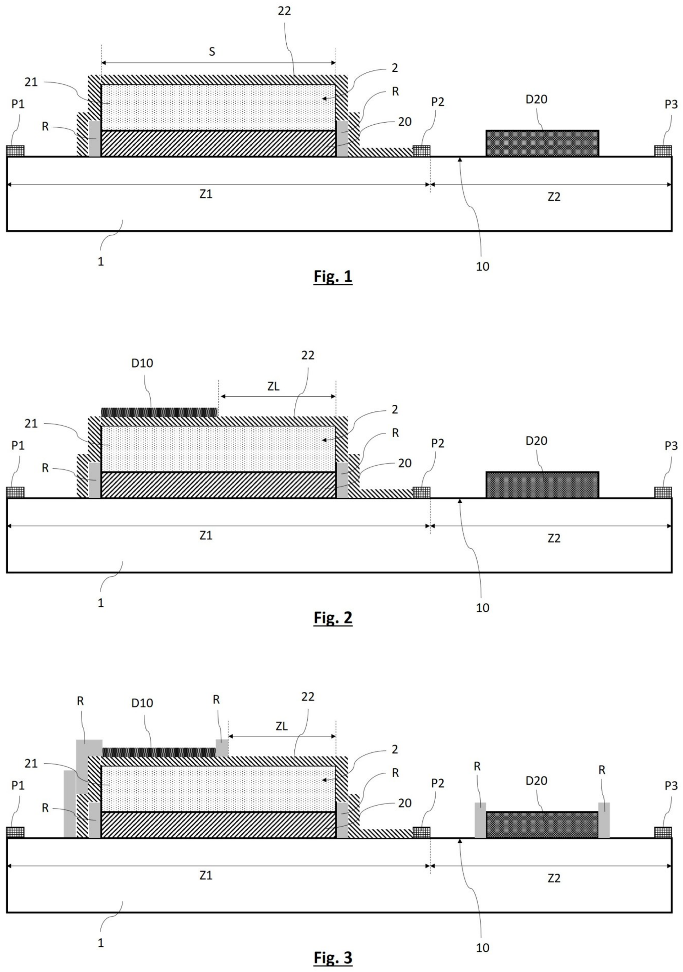

Figure 1 est une vue schématique en coupe illustrant un substrat sur lequel est formé un dispositif électroluminescent et une première électrode du photodétecteur de mesure.

Figure 2 est une vue schématique en coupe illustrant la formation d'une première électrode du photodétecteur de référence sur une partie de la surface de sortie du dispositif électroluminescent.

Figure 3 est une vue schématique en coupe, illustrant la formation d'une résine électriquement isolante.

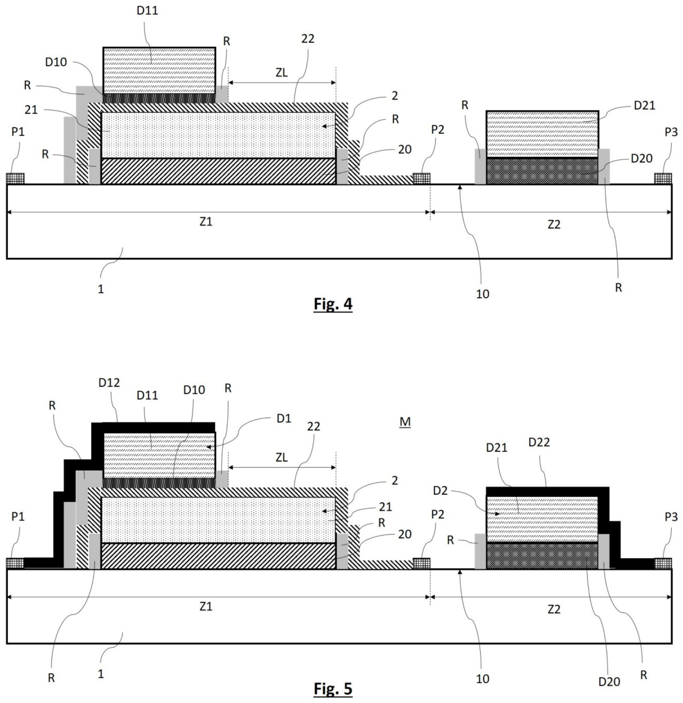

Figure 4 est une vue schématique en coupe, illustrant la formation d'empilements de couches, comprenant chacun une couche absorbante, pour former le photodétecteur de référence et le photodétecteur de mesure.

Figure 5 est une vue schématique en coupe, illustrant la formation d'une seconde électrode du photodétecteur de référence et du photodétecteur de mesure.

Il est à noter que les dessins décrits ci-avant sont schématiques, et ne sont pas nécessairement à l'échelle par souci de lisibilité et pour en simplifier leur compréhension. Les coupes sont effectuées selon la normale à la surface du substrat destinée à être orientée vers le milieu à analyser.

### Exposé détaillé des modes de réalisation

Les éléments identiques ou assurant la même fonction porteront les mêmes références pour les différents modes de réalisation, par souci de simplification.

### Système de mesure

Un objet de l'invention est un système de mesure spectroscopique à réflectance diffuse d'un milieu M à analyser, comportant :

- un substrat 1, comprenant une surface 10 destinée à être orientée vers le milieu M à analyser, la surface 10 présentant des première et deuxième zones Z1, Z2 adjacentes ;
- un dispositif électroluminescent 2, agencé sur la première zone Z1 de la surface 10 du substrat 1, et configuré pour émettre un rayonnement lumineux dans une gamme de longueurs d'onde ; le dispositif électroluminescent 2 possédant une surface de sortie S par laquelle est transmis le rayonnement lumineux vers le milieu M à analyser ;
- un premier photodétecteur D1, dit de référence, agencé sur une partie de la surface de sortie S du dispositif électroluminescent 2 de manière à recevoir une partie du rayonnement lumineux et de sorte que la surface de sortie S conserve une zone libre ZL ;
- un deuxième photodétecteur D2, dit de mesure, agencé sur la deuxième zone Z2 de la surface 10 du substrat 1 pour recevoir le rayonnement lumineux transmis par la zone libre ZL de la surface de sortie S du dispositif électroluminescent 2 et réfléchi de manière diffuse par le milieu M à analyser.

### Substrat

Le substrat 1 comprend une surface 10, de préférence plane, destinée à être orientée vers le milieu M à analyser. En d'autres termes, la surface 10 est destinée à faire face au milieu M à analyser. La surface 10 présente des première et deuxième zones adjacentes Z1, Z2.

Selon un premier mode de réalisation, le substrat 1 est réalisé dans un matériau réfléchissant dans la gamme de longueurs d'onde. Lorsque la gamme de longueurs d'onde est le domaine visible, le substrat 1 peut être réalisé dans un matériau choisi parmi le silicium, un métal recouvert d'un isolant électrique, un matériau souple de type polymère (e.g. le polyméthacrylate de méthyle PMMA) recouvert d'une couche métallique réfléchissante et d'un isolant électrique.

Selon un deuxième mode de réalisation, le substrat 1 est réalisé dans un matériau non-réfléchissant (e.g. transparent) dans la gamme de longueurs d'onde. Lorsque la gamme de longueurs d'onde est le domaine visible, le substrat 1 peut être réalisé dans un matériau choisi parmi un verre, un matériau souple de type polymère (e.g. le polyméthacrylate de méthyle PMMA).

A titre d'exemple non limitatif, le substrat 1 peut présenter une épaisseur comprise entre 50 μm et 1 mm.

### Dispositif électroluminescent

Le dispositif électroluminescent 2 est agencé sur la première zone Z1 de la surface 10 du substrat 1. Le dispositif électroluminescent 2 est configuré pour émettre un rayonnement lumineux dans une gamme de longueurs d'onde. La gamme de longueurs d'onde peut être le domaine visible [380 nm, 850 nm], permettant notamment d'analyser le taux d'oxygénation dans la peau et la mesure d'onde de pouls. La gamme de longueurs d'onde peut être le domaine infrarouge, par exemple [900 nm, 1600 nm], permettant notamment d'analyser le taux de glycémie et le taux d'hydratation.

Le dispositif électroluminescent 2 possède une surface de sortie S par laquelle le rayonnement lumineux émis est transmis vers le milieu M à analyser. A titre d'exemple non limitatif, la surface de sortie S peut présenter une aire, notée « $A_S$ », comprise entre 6400 μm² et 14400 μm², de préférence comprise entre 8100 μm² et 12100 μm².

Le dispositif électroluminescent 2 comporte avantageusement au moins une diode électroluminescente organique. Toutefois, il est possible d'envisager au moins une diode électroluminescente inorganique comme dispositif électroluminescent 2.

Selon un mode de réalisation, le dispositif électroluminescent 2 comporte successivement :

- une première électrode 20, agencée sur la première zone de la surface du substrat ;
- un empilement 21 de couches, comprenant une couche émissive configurée pour émettre le rayonnement lumineux dans la gamme de longueurs d'onde ;
- une seconde électrode 22, transparente dans la gamme de longueurs d'onde, et présentant une surface définissant la surface de sortie S du dispositif électroluminescent 2.

A titre d'exemple non limitatif, l'empilement 21 de couches peut comporter :

- une couche d'injection de charges, par exemple réalisée en Dipyrazino[2,3-f:2',3'-h]quinoxaline-2,3,6,7,10,11-hexacarbonitrile ;
- une couche de transport de charges, par exemple réalisée en 2,2',7,7'-Tetra(N,N-di-p-tolyl)amino-9,9-spirobifluorene ;
- une couche de blocage de charges, par exemple réalisée en N,N'-Bis(naphthalen-1-yl)-N,N'-bis(phenyl)-benzidine ;
- une couche émissive, choisie en fonction de la gamme de longueurs d'onde ;
- une couche de blocage de charges, par exemple réalisée en N,N'-Bis(3-methylphenyl)-N,N'-bis(phenyl)-benzidine ;
- une couche de transport de charges, par exemple réalisée en 4,7-Diphenyl-1,10-phenanthroline ;
- une couche d'injection de charges, par exemple réalisée en LiF.

### Photodétecteur de référence

Le premier photodétecteur D1 est agencé sur une partie de la surface de sortie S du dispositif électroluminescent 2 de sorte que la surface de sortie S conserve une zone libre ZL. La zone libre ZL de la surface de sortie S transmet une partie du rayonnement lumineux émis par le dispositif électroluminescent 2 vers le milieu M à analyser. L'autre partie du rayonnement lumineux émis par le dispositif électroluminescent 2 est reçu par le premier photodétecteur D1.

Le premier photodétecteur D1 comporte avantageusement au moins une photodiode organique.

Selon un mode de réalisation, le premier photodétecteur D1 comporte successivement :

- une première électrode D10, transparente dans la gamme de longueurs d'ondes, et agencée sur une partie de la surface de sortie S du dispositif électroluminescent 2 ;
- un empilement D11 de couches, comprenant une couche absorbante configurée pour absorber le rayonnement lumineux dans la gamme de longueurs d'onde ;
- une seconde électrode D12, réfléchissante dans la gamme de longueurs d'onde.

A titre d'exemple non limitatif, l'empilement D11 de couches peut comporter :

- une couche de transport de charges, par exemple réalisée en 2,2',7,7'-Tetra(N,N-di-p-tolyl)amino-9,9-spirobifluorene ;
- une couche de blocage de charges, par exemple réalisée en N,N'-Bis(naphthalen-1-yl)-N,N'-bis(phenyl)-benzidine ;
- une couche absorbante, photosensible, choisie en fonction de la gamme de longueurs d'onde, par exemple réalisée dans un mélange ZnPc : C60 pour le domaine visible ;
- une couche de blocage de charges, par exemple réalisée en N,N'-Bis(3-methylphenyl)-N,N'-bis(phenyl)-benzidine ;
- une couche de transport de charges, par exemple réalisée en 4,7-Diphenyl-1,10-phenanthroline.

La première électrode D10 du premier photodétecteur D1 est agencé directement sur une partie de la surface de sortie S du dispositif électroluminescent de sorte que la première électrode D10 du premier photodétecteur D1 est en contact avec la seconde électrode 22 du dispositif électroluminescent 2.

La première électrode D10 du premier photodétecteur D1 est avantageusement agencée sur une partie latérale de la surface de sortie S du dispositif électroluminescent 2.

La première électrode D10 du premier photodétecteur D1 occupe une aire, notée « $A_P$ », vérifiant avantageusement :

$$A_P = \frac{A_S}{n}$$

où « n » est un entier naturel supérieur ou égal à 2.

Lorsque l'aire de la surface de sortie S (« $A_S$ ») est comprise entre 6400 $\mu m^2$ et 14400 $\mu m^2$, de préférence comprise entre 8100 $\mu m^2$ et 12100 $\mu m^2$, alors « n » est avantageusement égal à 2.

### Photodétecteur de mesure

Le deuxième photodétecteur D2 est agencé sur la deuxième zone Z2 de la surface 10 du substrat 1 pour recevoir le rayonnement lumineux transmis par la zone libre ZL de la surface de sortie S du dispositif électroluminescent 2, puis réfléchi de manière diffuse par le milieu M à analyser.

Le deuxième photodétecteur D2 comporte avantageusement au moins une photodiode organique.

Selon un mode de réalisation, le deuxième photodétecteur D2 comporte successivement :

- une première électrode D20, réfléchissante dans la gamme de longueurs d'ondes, et agencée sur la deuxième zone Z2 de la surface 10 du substrat 1 ;
- un empilement D21 de couches, comprenant une couche absorbante configurée pour absorber le rayonnement lumineux dans la gamme de longueurs d'onde ;
- une seconde électrode D22, transparente dans la gamme de longueurs d'onde.

Plus précisément, la couche absorbante de l'empilement D21 de couches du deuxième photodétecteur D2 est configurée pour absorber le rayonnement lumineux réfléchi de manière diffuse par le milieu M à analyser.

A titre d'exemple non limitatif, l'empilement D21 de couches peut comporter :

- une couche de transport de charges, par exemple réalisée en 2,2',7,7'-Tetra(N,N-di-p-tolyl)amino-9,9-spirobifluorene ;
- une couche de blocage de charges, par exemple réalisée en N,N'-Bis(naphthalen-1-yl)-N,N'-bis(phenyl)-benzidine ;
- une couche absorbante, photosensible, choisie en fonction de la gamme de longueurs d'onde, par exemple réalisée dans un mélange ZnPc : C60 pour le domaine visible ;
- une couche de blocage de charges, par exemple réalisée en N,N'-Bis(3-methylphenyl)-N,N'-bis(phenyl)-benzidine ;
- une couche de transport de charges, par exemple réalisée en 4,7-Diphenyl-1,10-phenanthroline.

### Milieu à analyser

A titre d'exemple non limitatif, le milieu M à analyser est un tissu biologique de type peau comportant des chromophores. En particulier, les mélanosomes et les cellules sanguines de la peau agissent comme des centres diffuseurs du rayonnement lumineux, dispersés dans le milieu M à analyser. Par exemple, l'homme du métier sait analyser la couleur de la peau (carnation) en fonction des mesures de réflectance diffuse à partir de modèles optiques utilisant la théorie de Mie.

### Encapsulation et isolation électrique

De manière avantageuse, les premier et deuxième photodétecteurs D1, D2 sont recouverts d'une couche d'encapsulation conçue pour les protéger contre le milieu M à analyser, l'air, l'humidité etc. La couche d'encapsulation est transparente dans la gamme de longueurs d'onde. La couche d'encapsulation peut de type multicouche. La couche d'encapsulation est avantageusement imperméable à l'eau, la vapeur d'eau, le dioxygène, une agression extérieur comme par exemple la sueur. A titre d'exemples non limitatifs, la couche d'encapsulation peut comporter au moins un matériau choisi parmi SiO, TiO$_2$, Al$_2$O$_3$, le parylène.

Le système selon l'invention comporte avantageusement une résine R électriquement isolante agencée pour isoler :

- les première et seconde électrodes 20, 22 du dispositif électroluminescent 2 entre elles ;
- les première et seconde électrodes D10, D12 du premier photodétecteur D1 entre elles ;
- les première et seconde électrodes D20, D22 du deuxième photodétecteur D2 entre elles.

**Procédé de fabrication**

Un objet de l'invention est un procédé de fabrication d'un système de mesure spectroscopique à réflectance diffuse d'un milieu M à analyser, comportant les étapes :

a) utiliser un substrat 1, comprenant une surface 10 destinée à être orientée vers le milieu M à analyser, la surface 10 présentant des première et deuxième zones adjacentes Z1, Z2 ;
b) former un dispositif électroluminescent 2 sur la première zone Z1 de la surface 10 du substrat 1, le dispositif électroluminescent 2 étant configuré pour émettre un rayonnement lumineux dans une gamme de longueurs d'onde ; le dispositif électroluminescent 2 possédant une surface de sortie S par laquelle est transmis le rayonnement lumineux vers le milieu M à analyser ;
c) former un premier photodétecteur D1, dit de référence, sur une partie de la surface de sortie S du dispositif électroluminescent 2 de manière à recevoir une partie du rayonnement lumineux et de sorte que la surface de sortie S conserve une zone libre ZL ;
d) former un deuxième photodétecteur D2, dit de mesure, sur la deuxième zone Z2 de la surface 10 du substrat 1 pour recevoir le rayonnement lumineux transmis par la zone libre ZL de la surface de sortie S du dispositif électroluminescent 2 et réfléchi de manière diffuse par le milieu M à analyser.

*Etape a)*

L'étape a) consiste à utiliser un substrat 1, comprenant une surface 10 destinée à être orientée vers le milieu M à analyser. En d'autres termes, la surface 10 est destinée à faire face au milieu M à analyser. La surface 10 présente des première et deuxième zones adjacentes Z1, Z2. Les caractéristiques techniques du substrat 1 décrites précédemment s'appliquent à l'étape a) du procédé selon l'invention.

*Etape b)*

L'étape b) consiste à former un dispositif électroluminescent 2 sur la première zone Z1 de la surface 10 du substrat 1. Le dispositif électroluminescent 2 est configuré pour émettre un rayonnement lumineux dans une gamme de longueurs d'onde. Le dispositif électroluminescent 2 possède une surface de sortie S par laquelle est transmis le rayonnement lumineux vers le milieu M à analyser. Les caractéristiques techniques du dispositif électroluminescent 2 décrites précédemment s'appliquent à l'étape b) du procédé selon l'invention.

L'étape b) peut comporter les étapes :

b$_1$) former une première électrode 20 sur la première zone Z1 de la surface 10 du substrat 1 ;
b$_2$) former un empilement 21 de couches sur la première électrode 20, l'empilement 21 comprenant une couche émissive configurée pour émettre le rayonnement lumineux dans la gamme de longueurs d'onde ;
b$_3$) former une seconde électrode 22, transparente dans la gamme de longueurs d'onde, sur l'empilement 21 de couches, et présentant une surface définissant la surface de sortie S du dispositif électroluminescent 2.

*Etape c)*

L'étape c) consiste à former un premier photodétecteur D1, dit de référence, sur une partie de la surface de sortie S du dispositif électroluminescent 2 de sorte que la surface de sortie S conserve une zone libre ZL. La zone libre ZL de la surface de sortie S transmet une partie du rayonnement lumineux émis par le dispositif électroluminescent 2 vers le milieu M à analyser. L'autre partie du rayonnement lumineux émis par le dispositif électroluminescent 2 est reçu par le premier photodétecteur D1.

Les caractéristiques techniques du premier photodétecteur D1 décrites précédemment s'appliquent à l'étape c) du procédé selon l'invention.

*Etape d)*

L'étape d) consiste à former un deuxième photodétecteur D2, dit de mesure, sur la deuxième zone Z2 de la surface 10 du substrat 1. Le deuxième photodétecteur D2 est agencé sur la deuxième zone Z2 de la surface 10 du substrat 1 pour recevoir le rayonnement lumineux transmis par la zone libre ZL de la surface de sortie S du dispositif électroluminescent 2, puis réfléchi de manière diffuse par le milieu M à analyser.

Les caractéristiques techniques du deuxième photodétecteur D2 décrites précédemment s'appliquent à l'étape d) du procédé selon l'invention.

Les étapes c) et d) sont avantageusement concomitantes. Les étapes c) et d) concomitantes comportent avantageusement les étapes :

- former une première électrode D10, D20 sur une partie de la surface de sortie S du dispositif électroluminescent 2 ainsi que sur la deuxième zone Z2 de la surface 10 du substrat 1 ;

- former un empilement D11, D21 de couches, comprenant une couche absorbante configurée pour absorber le rayonnement lumineux dans la gamme de longueurs d'onde, sur la première électrode D10 du premier photodétecteur D1 ainsi que sur la première électrode D20 du deuxième photodétecteur D2 ;
- former une seconde électrode D12, D22 sur l'empilement D11 de couches du premier photodétecteur D1 ainsi que sur l'empilement D21 de couches du deuxième photodétecteur D2.

### *Plots de contact*

L'étape b) est avantageusement précédée des étapes :

- former des premiers plots P1 de contact, agencés sur la première zone Z1 de la surface 10 du substrat 1 pour une reprise de contact électrique de la seconde électrode 22 du dispositif électroluminescent 2 ;
- former des deuxièmes plots P2 de contact, agencés sur la première zone Z1 de la surface 10 du substrat 1 pour une reprise de contact électrique de la seconde électrode D12 du premier photodétecteur D1 ;
- former des troisièmes plots P3 de contact, agencés sur la deuxième zone Z2 de la surface 10 du substrat 1 pour une reprise de contact électrique de la seconde électrode D22 du deuxième photodétecteur D2.

La seconde électrode 22 du dispositif électroluminescent 2 formée lors de l'étape b₃) s'étend avantageusement sur la première zone Z1 de la surface 10 du substrat 1 de manière à être électriquement connectée aux premiers plots P1 de contact.

De manière avantageuse, la seconde électrode D12 du premier photodétecteur D1 et la seconde électrode D22 du deuxième photodétecteur D2 formées lors des étapes c) et d) concomitantes s'étendent respectivement sur les première et deuxième zones Z1, Z2 de la surface 10 du substrat 1, de manière à être respectivement électriquement connectées aux deuxièmes et troisièmes plots P2, P3 de contact.

Selon une alternative, les deuxièmes plots P2 de contact et les troisièmes plots P3 de contact peuvent être électriquement connectés entre eux. Le cas échéant, la seconde électrode D12 du premier photodétecteur D1 et la seconde électrode D22 du deuxième photodétecteur D2 peuvent être communes.

L'invention ne se limite pas aux modes de réalisation exposés. L'homme du métier est mis à même de considérer leurs combinaisons techniquement opérantes, et de leur substituer des équivalents.

## Revendications

1. Système de mesure spectroscopique à réflectance diffuse d'un milieu (M) à analyser, comportant :

    - un substrat (1), comprenant une surface (10) destinée à être orientée vers le milieu (M) à analyser, la surface (10) présentant des première et deuxième zones (Z1, Z2) adjacentes ;
    - un dispositif électroluminescent (2), agencé sur la première zone (Z1) de la surface (10) du substrat (1), et configuré pour émettre un rayonnement lumineux dans une gamme de longueurs d'onde ; le dispositif électroluminescent (2) possédant une surface de sortie (S) par laquelle est transmis le rayonnement lumineux vers le milieu (M) à analyser, la surface de sortie (S) possède une aire, notée « A$_S$ » ;
    - un premier photodétecteur (D1), dit de référence, agencé sur une partie de la surface de sortie (S) du dispositif électroluminescent (2) de manière à recevoir une partie du rayonnement lumineux et de sorte que la surface de sortie (S) conserve une zone libre (ZL) ; le premier photodétecteur (D1) comporte successivement :

        une première électrode (D10), transparente dans la gamme de longueurs d'ondes, et agencée sur une partie de la surface de sortie (S) du dispositif électroluminescent (2), la première électrode (D10) du premier photodétecteur (D1) occupe une aire, notée « A$_P$ », vérifiant :

$$A_P = \frac{A_S}{n}$$

        où « n » est un entier naturel supérieur ou égal à 2 ;
        un empilement (D11) de couches, comprenant une couche absorbante configurée pour absorber le rayonnement lumineux dans la gamme de longueurs d'onde ;
        une seconde électrode (D12), réfléchissante dans la gamme de longueurs d'onde ;

    - un deuxième photodétecteur (D2), dit de mesure, agencé sur la deuxième zone (Z2) de la surface (10) du substrat (1) pour recevoir le rayonnement lumineux transmis par la zone libre (ZL) de la surface de sortie (S) du dispositif électroluminescent (2) et réfléchi de manière diffuse par le milieu (M) à analyser.

2. Système selon la revendication 1, dans lequel le dispositif électroluminescent (2) comporte au moins une diode électroluminescente organique.

**3.** Système selon la revendication 1 ou 2, dans lequel le premier photodétecteur (D1) comporte au moins une photodiode organique.

**4.** Système selon l'une des revendications 1 à 3, dans lequel le deuxième photodétecteur (D2) comporte au moins une photodiode organique.

**5.** Système selon l'une des revendications 1 à 4, dans lequel le dispositif électroluminescent (2) comporte successivement :

- une première électrode (20), agencée sur la première zone (Z1) de la surface (10) du substrat (1) ;
- un empilement (21) de couches, comprenant une couche émissive configurée pour émettre le rayonnement lumineux dans la gamme de longueurs d'onde ;
- une seconde électrode (22), transparente dans la gamme de longueurs d'onde, et présentant une surface définissant la surface de sortie (S) du dispositif électroluminescent (2).

**6.** Système selon l'une des revendications 1 à 5, dans lequel la première électrode (D10) du premier photodétecteur (D1) est agencée sur une partie latérale de la surface de sortie (S).

**7.** Système selon l'une des revendications 1 à 6, dans lequel :

- l'aire de la surface de sortie (S) est comprise entre 6400 $\mu$m² et 14400 $\mu$m², de préférence comprise entre 8100 $\mu$m² et 12100 $\mu$m² ;
- « n » est égal à 2.

**8.** Système selon l'une des revendications 1 à 7, dans lequel le substrat (1) est réalisé dans un matériau réfléchissant dans la gamme de longueurs d'onde.

**9.** Procédé de fabrication d'un système de mesure spectroscopique à réflectance diffuse d'un milieu (M) à analyser, comportant les étapes :

a) utiliser un substrat (1), comprenant une surface (10) destinée à être orientée vers le milieu (M) à analyser, la surface (10) présentant des première et deuxième zones (Z1, Z2) adjacentes ;
b) former un dispositif électroluminescent (2) sur la première zone (Z1) de la surface (10) du substrat (1), le dispositif électroluminescent (2) étant configuré pour émettre un rayonnement lumineux dans une gamme de longueurs d'onde ; le dispositif électroluminescent (2) possédant une surface de sortie (S) par laquelle est transmis le rayonnement lumineux vers le milieu (M) à analyser, la surface de sortie (S) possède une aire, notée « A_S » ;
c) former un premier photodétecteur (D1), dit de référence, sur une partie de la surface de sortie (S) du dispositif électroluminescent (2) de manière à recevoir une partie du rayonnement lumineux et de sorte que la surface de sortie (S) conserve une zone libre (ZL) ; le premier photodétecteur (D1) comporte successivement :

une première électrode (D10), transparente dans la gamme de longueurs d'ondes, et agencée sur une partie de la surface de sortie (S) du dispositif électroluminescent (2), la première électrode (D10) du premier photodétecteur (D1) occupe une aire, notée « A_P », vérifiant :

$$A_P = \frac{A_S}{n}$$

où « n » est un entier naturel supérieur ou égal à 2 ;
un empilement (D11) de couches, comprenant une couche absorbante configurée pour absorber le rayonnement lumineux dans la gamme de longueurs d'onde ;
une seconde électrode (D12), réfléchissante dans la gamme de longueurs d'onde ;

d) former un deuxième photodétecteur (D2), dit de mesure, sur la deuxième zone (Z2) de la surface (10) du substrat (1) pour recevoir le rayonnement lumineux transmis par la zone libre (ZL) de la surface de sortie (S) du dispositif électroluminescent (2) et réfléchi de manière diffuse par le milieu (M) à analyser.

**10.** Procédé selon la revendication 9, dans lequel les étapes c) et d) sont concomitantes.

**11.** Procédé selon la revendication 10, dans lequel l'étape b) comporte les étapes :

b_1) former une première électrode (20) sur la première zone (Z1) de la surface (10) du substrat (1) ;
b_2) former un empilement (21) de couches sur la première électrode (20), l'empilement (21) comprenant une couche émissive configurée pour émettre le rayonnement lumineux dans la gamme de longueurs d'onde ;
b_3) former une seconde électrode (22), transparente dans la gamme de longueurs d'onde, sur l'empilement (21) de couches, et présentant une surface définissant la surface de sortie (S) du dispositif électroluminescent (2) ;

procédé dans lequel les étapes c) et d) concomitantes comportent les étapes :

- former une première électrode (D10, D20) sur une partie de la surface de sortie (S) du dispositif électroluminescent (2) ainsi que sur la deuxième zone (Z2) de la surface (10) du substrat (1) ;
- former un empilement (D11, D21) de couches, comprenant une couche absorbante configurée pour absorber le rayonnement lumineux dans la gamme de longueurs d'onde, sur la première électrode (D10) du premier photodétecteur (D1) ainsi que sur la première électrode (D20) du deuxième photodétecteur (D2) ;
- former une seconde électrode (D12, D22) sur l'empilement (D11) de couches du premier photodétecteur (D1) ainsi que sur l'empilement (D21) de couches du deuxième photodétecteur (D2).

12. Procédé selon la revendication 11, dans lequel l'étape b) est précédée des étapes :

- former des premiers plots (P1) de contact, agencés sur la première zone (Z1) de la surface (10) du substrat (1) pour une reprise de contact électrique de la seconde électrode (22) du dispositif électroluminescent (2) ;
- former des deuxièmes plots (P2) de contact, agencés sur la première zone (Z1) de la surface (10) du substrat (1) pour une reprise de contact électrique de la seconde électrode (D12) du premier photodétecteur (D1) ;
- former des troisièmes plots (P3) de contact, agencés sur la deuxième zone (Z2) de la surface (10) du substrat (1) pour une reprise de contact électrique de la seconde électrode (D22) du deuxième photodétecteur (D2) ;

procédé dans lequel :

- la seconde électrode (22) du dispositif électroluminescent (2) formée lors de l'étape $b_3$) s'étend sur la première zone (Z1) de la surface (10) du substrat (1) de manière à être électriquement connectée aux premiers plots (P1) de contact ;
- la seconde électrode (D12) du premier photodétecteur (D1) et la seconde électrode (D22) du deuxième photodétecteur (D2) formées lors des étapes c) et d) concomitantes s'étendent respectivement sur les première et deuxième zones (Z1, Z2) de la surface (10) du substrat (1), de manière à être respectivement électriquement connectées aux deuxièmes et troisièmes plots (P2, P3) de contact.

**Fig. 1**

**Fig. 2**

**Fig. 3**

**Fig. 4**

**Fig. 5**

Europäisches Patentamt
European Patent Office
Office européen des brevets

**RAPPORT DE RECHERCHE EUROPEENNE**

Numéro de la demande

EP 25 20 7901

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | US 2023/118120 A1 (EBSTEIN STEVEN M [US] ET AL) 20 avril 2023 (2023-04-20) <br> * abrégé; figure 3 * <br> * alinéas [0008] - [0009] * <br> ----- | 1-12 | INV. <br> G01N21/47 <br> A61B5/00 <br> A61B5/1455 |
| Y | US 2022/057324 A1 (BAUMGARTNER ANDREAS [DE] ET AL) 24 février 2022 (2022-02-24) <br> * abrégé; figures * <br> * alinéas [0027] - [0047], [0050] * <br> ----- | 1-12 | |
| Y | US 2009/074017 A1 (CHOI JIN-KYUNG [KR]) 19 mars 2009 (2009-03-19) <br> * alinéas [0051] - [0056], [0058]; figures 2-3 * <br> ----- | 1-12 | |
| Y | US 2006/192088 A1 (KOYAMA TOMOKO [JP]) 31 août 2006 (2006-08-31) <br> * alinéas [0060], [0073], [0075] - [0076], [0081]; figure 7 * <br> ----- | 1-12 | |
| A | US 4 770 536 A (GOLBERSTEIN MOSHE [US]) 13 septembre 1988 (1988-09-13) <br> * colonne 3, lignes 10-52; figures 1,4 * <br> ----- | 1-12 | **DOMAINES TECHNIQUES RECHERCHES (IPC)** <br><br> G01N <br> A61B |

Le présent rapport a été établi pour toutes les revendications

| Lieu de la recherche | Date d'achèvement de la recherche | Examinateur |
|---|---|---|
| Munich | 11 février 2026 | Riblet, Philippe |

**ANNEXE AU RAPPORT DE RECHERCHE EUROPEENNE
RELATIF A LA DEMANDE DE BREVET EUROPEEN NO.**

EP 25 20 7901

La présente annexe indique les membres de la famille de brevets relatifs aux documents  brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

11-02-2026

| Document brevet cité au rapport de recherche | Date de publication | Membre(s) de la famille de brevet(s) | Date de publication |
|---|---|---|---|
| US 2023118120 A1 | 20-04-2023 | EP 4419891 A1 | 28-08-2024 |
| | | US 2023118120 A1 | 20-04-2023 |
| | | WO 2023069646 A1 | 27-04-2023 |
| US 2022057324 A1 | 24-02-2022 | CN 113574364 A | 29-10-2021 |
| | | DE 102019203318 A1 | 17-09-2020 |
| | | EP 3938761 A1 | 19-01-2022 |
| | | JP 2022524603 A | 09-05-2022 |
| | | KR 20210135574 A | 15-11-2021 |
| | | US 2022057324 A1 | 24-02-2022 |
| | | WO 2020182388 A1 | 17-09-2020 |
| US 2009074017 A1 | 19-03-2009 | CN 101394063 A | 25-03-2009 |
| | | KR 20090028979 A | 20-03-2009 |
| | | US 2009074017 A1 | 19-03-2009 |
| US 2006192088 A1 | 31-08-2006 | JP 4019285 B2 | 12-12-2007 |
| | | JP 2006216846 A | 17-08-2006 |
| | | US 2006192088 A1 | 31-08-2006 |
| US 4770536 A | 13-09-1988 | AUCUN | |

EPO FORM P0460

Pour tout renseignement concernant cette annexe : voir Journal Officiel de l'Office européen des brevets, No.12/82

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2023094887 A1